# EUROPEAN PATENT APPLICATION

(11) **EP 4 760 251 A1**
(43) Date of publication of application: **17.06.2026**
(21) Application number: 25223227.7
(22) Date of filing: 15.12.2025
(51) Int. Cl.: G01N 27/327, G01N 33/543

(54) **METHOD FOR PRODUCING PRODUCT COMPRISING CARBON ALLOTROPE LAYER, PRODUCT AND USE OF PRODUCT**

(30) Priority: 16.12.2024 FI 20246467
(71) Applicant: Canatu Finland Oy, 01720 Vantaa (FI)
(72) Inventor: Dulay, Samuel, 01720 Vantaa (FI); Rantataro, Samuel, 01720 Vantaa (FI); Joon, Narender, 01720 Vantaa (FI)
(74) Representative: Primrose Oy

(57) **Abstract**

The disclosure relates to a method for producing a product comprising a substrate and a carbon allotrope layer connected to a surface of the substrate. The method comprises providing a substrate, generating functional groups on the surface of the substrate, bonding a first molecule to at least one of the functional groups through covalent bonding, and applying a carbon allotrope layer on the surface of the substrate after bonding the first molecule to at least one of the functional groups.

The disclosure relates also to a product comprising a substrate having a surface comprising functional groups, a first molecule bonded to at least one of the functional groups through covalent bonding, and a carbon allotrope layer; wherein the carbon allotrope layer is connected to the surface of the substrate with the first molecule.

## Description

### FIELD OF THE DISCLOSURE

The present disclosure relates to a method for producing a product comprising carbon allotrope layer, and more particularly to a method for producing a product comprising a substrate and a carbon allotrope layer connected to the surface of the substrate.

The present disclosure relates also to a product comprising a carbon allotrope layer, and more particularly to a product comprising a carbon allotrope layer connected to surface of a substrate.

The present disclosure further concerns the use of the product in a biosensor.

### BACKGROUND OF THE DISCLOSURE

Carbon allotropes, such as carbon nanotubes are used in many applications. For example, a carbon nanotube (CNT) film connected to a substrate can be used as a sensitive transducer in biosensors in *in-vitro* diagnostics applications. When connecting the CNT film on the substrate, a thin film of adhesive, such as organosilicate, can be used.

One of the problems associated with the prior art is that connecting, such as adhering, the CNT film on the substrate may be difficult, especially if the substrate is, for example, polypropylene or borosilicate glass. Even a thin layer of adhesive may compromise the electrochemical performance of the transducer. This weak interfacial bonding often compromises the mechanical integrity and long-term stability of the CNT.

Further, the inherently hydrophobic nature of pristine CNTs severely restricts their direct interaction with polar substances. This hydrophobicity significantly impedes their interaction with essential biomolecules (such as proteins, DNA, and enzymes) and various ions, which are ubiquitous in biological and electrochemical systems.

### BRIEF DESCRIPTION OF THE DISCLOSURE

An object of the present disclosure is to provide a method for producing a product comprising a carbon allotrope layer, and a product solving above problems.

The object of the disclosure is achieved by the method and the product which are characterized by what is stated in the independent claims. The preferred embodiments of the disclosure are disclosed in the dependent claims.

The disclosure is based on the idea of providing a method for producing a product comprising a substrate and a carbon allotrope layer connected to a surface of the substrate, wherein the method comprises
- providing a substrate,
- generating functional groups on the surface of the substrate, wherein the functional groups are selected from a group consisting of hydroxyl groups, amine groups and carboxyl groups,
- bonding a first molecule to at least one of the functional groups through covalent bonding, and
- applying a carbon allotrope layer on the surface of the substrate after bonding the first molecule to at least one of the functional groups.

The disclosure is also based on the idea of providing a product, wherein the product comprises
- a substrate having a surface comprising functional groups, wherein the functional groups are selected from a group consisting of hydroxyl groups, amine groups and carboxyl groups,
- a first molecule bonded to at least one of the functional groups on the surface of the substrate through covalent bonding, and
- a carbon allotrope layer comprising a first surface and a second surface opposite the first surface;
wherein the first surface of the carbon allotrope layer is connected to the surface of the substrate with the first molecule.

An advantage of the disclosure is that generating the functional groups on the surface of the substrate and bonding the first molecule to the functional groups creates a single layer of first molecule on the surface of the substrate to connect the carbon allotrope layer to the surface of the substrate. In other words, this provides the connecting layer, such as the adhesive layer, with the minimum possible thickness, which in turn has the minimum effect on the electrochemical performance of the transducer.

### DETAILED DESCRIPTION OF THE DISCLOSURE

### Method

The disclosure relates to a method for producing a product comprising a substrate having a surface and a carbon allotrope layer connected to the surface of the substrate. For example, if the substrate is in the form of a sheet having two opposite surfaces, the carbon allotrope layer is connected to a single surface of the substrate or to both surfaces of the substrate. If the substrate has some other form, the carbon allotrope layer may be connected to the whole surface or a part of the surface.

For example, the product can be used as a platform for a sensitive transducer for a sensing probe in a biosensor and in *in-vitro* diagnostics applications.

### Providing substrate

The method comprises providing a substrate having a surface. The substrate is made of solid material. The surface of the substrate is for example silicon (Si), silicon wafer, silicon dioxide (SiO₂) on silicon, quartz (SIO₂), fused quartz, aluminium Oxide (Al₂O₃), sapphire (Al₂O₃), titanium (Ti), titanium dioxide (TiO₂), glass, borofloat glass, polytetrafluoroethylene (PTFE), polyimide (PI), polycarbonate (PC), polycarbonate-polymethyl methacrylate (PC/PMMA) composite, carbon-based substrate, such as graphene, highly ordered pyrolytic graphite (HOPG), magnesium oxide (MgO), zirconium oxide (ZrO₂), or plastic film, such as polyethylene terephthalate (PET) or polystyrene (PS). For example, the substrate is in a plate form.

### Generating functional groups

The method comprises activating the surface of the substrate by generating functional groups on the surface of the substrate. The purpose of the functional groups is to enhance bonding of a further substance to the surface of the substrate. The functional groups are selected from a group consisting of hydroxyl groups (-OH), amine groups (-NH₂) and carboxyl groups (-COOH). In other words, the functional groups generated on the surface of the substrate are either hydroxyl groups, amine groups or carboxyl groups, or they can be any combination of those.

According to some embodiments, the functional groups are generated on the surface of the substrate by treating the surface of the substrate by physical treatment, e.g. by plasma treatment or corona treatment, or by chemical treatment, e.g. by acid treatment.

In the acid treatment, the surface of the substrate is exposed to the acid to form the functional groups. The acid treatment can be performed for example using acetic acid, maleic anhydride, citric acid, sulfuric acid or phosphoric acid.

### Bonding first molecule

The method comprises bonding a first molecule, or a primer molecule, such as a plurality of first molecules, to at least one of the functional groups through covalent bonding. In other words, the first molecule is applied on the activated surface of the substrate and allowed to bond with the functional groups. The first molecule may bond with a single functional group, or it may bond with a plurality of functional groups. In this context, term *"first molecule"* means at least one molecule of a first molecular substance.

The first molecule may adhere the carbon allotrope layer to the substrate.

Because the functional groups are highly reactive and therefore degrade easily, the first molecule is preferably applied on the activated surface in less than 3 h of the activation of the surface.

For example, the first molecule is a polymer. According to some embodiments, the first molecule is an organosilane, such as selected from a group consisting of trimethoxymethylsilane (TMMS), dimethoxydiphenylsilane (DMDPD) and 3-(triethoxysilyl)propylmalonic acid, or the first molecule is a molecule containing at least one primary amine group, such as selected from a group consisting of (3-aminopropyl)trimethoxysilane (APTMS), bis(3-trimethoxysilylpropyl)amine (BTMSPA), 1,4-butanediol, chitosan, preferably low molecular weight chitosan, ethylenediamine (EDA), hexamethylenediamine (HMD), melamine, m-phenylenediamine, p-phenylenediamine, piperazine, polyallylamine (PAAm), branched polyethyleneimine (PEI), poly[imino(1,6-dioxohexamethylene) iminohexamethylene] (nylon 66), polyvinylamine (PVAm) and (3-aminopropyl)triethoxysilane (APTES). Preferably, the first molecule is chitosan.

For example, the first molecule is applied as an aqueous solution comprising the first molecule. For example, the concentration of the first molecule in the aqueous solution is 2 to 5 % by weight. Preferably, the first molecule is applied on the activated surface of the substrate in an amount to saturate the functional groups. This is to maximize the number of first molecules bonding to the functional groups. For example, the aqueous solution comprising the first molecule is applied on the activated surface of the substrate in an excess amount and unbonded solution is then removed from the surface.

### Applying carbon allotrope layer

The method comprises applying a carbon allotrope layer on the surface of the substrate after bonding the first molecule to at least one of the functional groups.

For example, the carbon allotrope layer is a carbon nanotube layer or a graphene layer, such as a carbon nanotube film or a single-layer graphene film. The carbon allotrope layer may also be a vertically aligned carbon nanotube layer, i.e. a layer in which the carbon nanotubes are oriented parallel to the thickness of the layer. The carbon allotrope layer forms a mesh of carbon atoms bound to each other.

Preferably, the thickness of the carbon allotrope layer is 10 to 350 nm.

### Bonding tethering molecule

According to some embodiments, the method comprises bonding a tethering molecule to the first molecule bonded to at least one of the functional groups. In other words, the tethering molecule is applied on the surface of the substrate. As a result, the first molecule and the tethering molecule form a molecule chain comprising the first molecule and the tethering molecule. In this context, term *"tethering molecule"* means at least one molecule of a molecular substance that is able to bond with two separate molecules through covalent bonding.

Preferably, the tethering molecule is applied as an aqueous solution comprising the tethering molecule. The purpose of the tethering molecule may be to allow bonding a further substance to the first molecule via the tethering molecule.

The tethering molecule is preferably applied on the surface of the substrate in less than 2 h of bonding the first molecule. This is to reduce the bonding of the first molecule to something else than the tethering molecule.

According to some embodiments, the method comprises applying the tethering molecule on the carbon allotrope layer after applying the carbon allotrope layer on the surface of the substrate to bond the tethering molecule to the to the first molecule through the carbon allotrope layer. In this case, the tethering molecule bonds to the first molecule through the mesh formed by the carbon allotrope layer immobilizing the carbon allotrope layer on the surface of the substrate.

The tethering molecule may act as a hydrophilic agent, which infiltrates and surrounds the porous carbon allotrope network. This renders the external surfaces of the carbon allotrope layer hydrophilic while preserving the inherent pristine quality of the carbon allotrope layer. This enhances the electrochemical characteristics of the product without compromising the fundamental properties for its intended end-use.

According to some embodiments, when the first molecule is preferably an organosilane, such as 3-(triethoxysilyl)propylmalonic acid, the tethering molecule is 1-ethyl-3-(3-dimethylaminopropyl)carbodiimide (EDC) or N,N'-dicyclohexylcarbodiimide (DCC).

According to some embodiments, when the first molecule is preferably a molecule containing at least one primary amine group, the tethering molecule is glutaraldehyde (GA), N-γ-maleimidobutyryl-oxysuccinimide ester (GMBS) or an activated dicarboxylic acid, such as oxalic acid. In other words, the tethering molecule is selected from a group consisting of glutaraldehyde (GA), N-γ-maleimidobutyryl-oxysuccinimide ester (GMBS) and activated dicarboxylic acids, such as oxalic acid. GA, GMBS and activated dicarboxylic acids are able to conjugate with the first molecule through the at least one primary amine group in the first molecule, thus creating a strong bond with the first molecule. Preferably, the tethering molecule is glutaraldehyde (GA). GA acts as a hydrophilic agent. More preferably, the first molecule is chitosan, and the tethering molecule is GA.

According to some embodiments, the tethering molecule is applied on the surface of the substrate before applying the carbon allotrope layer on the surface of the substrate. Preferably, the tethering molecule is applied on the surface of the substrate in an amount to saturate the first molecule. This is to maximize the number of tethering molecules bonded to the first molecule. For example, if the first molecule is a molecule comprising primary amine groups and the tethering molecule is GA, GMBS or an activated dicarboxylic acid, the tethering molecule is added in such an amount that the primary amine groups are saturated with the tethering molecule. For example, the aqueous solution comprising the tethering molecule is applied on the surface of the substrate in an excess amount and unbonded solution is then removed from the surface.

Because the bonding of a tethering molecule being GA, GMBS or an activated dicarboxylic acid to a first molecule comprising at least one primary amine group utilizes the principles of click chemistry, the bonding takes place quickly. Therefore there is no need for the tethering molecule to remain in contact with the first molecule for an extended period of time.

### Applying second molecule

According to some embodiments, the method comprises, after bonding a tethering molecule to the first molecule and after applying the carbon allotrope layer on the surface of the substrate, applying a second molecule on the carbon allotrope layer. In this case, the tethering molecule is preferably bonded to the first molecule before applying the carbon allotrope layer on the surface of the substrate. In this context, term *"second molecule"* means at least one molecule of a second molecular substance.

The second molecule bonds to the tethering molecule through the carbon allotrope layer. In other words, the second molecule bonds to the tethering molecule through the mesh formed by the carbon allotrope layer. As a result, the first molecule, the tethering molecule and the second molecule form a molecule chain comprising the first molecule, the tethering molecule and the second molecule. Bonding of the second molecule to the tethering molecule through the carbon allotrope layer immobilizes the carbon allotrope layer on the surface of the substrate. In other words, bonding of the second molecule to the tethering molecule through the carbon allotrope layer forms a structure that utilizes mechanically interlocked molecular architecture (MIMA). As a result, the carbon allotrope layer cannot be removed from the surface of the substrate without breaking the covalent bonds of the molecule chain comprising the first molecule, the tethering molecule and the second molecule.

Carbon allotrope layer immobilized on the surface of the substrate reduces fouling and saturation of the electrode surfaces when the product is used as an electrode in a biosensor transducer. Fouling and saturation of the transducer surface leads often to surface insulation, poor performance and compromised sensitivity. This also facilitates better recognition of target analyte of interest because of a non-steric environment created by the underlying surface of the substrate covered by the porous surface of the carbon allotrope layer where it is attached.

For example, the second molecule is a polymer. According to some embodiments, the second molecule is a molecule containing at least one primary amine group, such as selected from a group consisting of polyallylamine (PAAm), poly(amino acid), chitosan, preferably low molecular weight chitosan, branched polyethyleneimine (PEI), poly(glutamic acid), poly(L-lysine) (PLL), polyvinylamine (PVAm), poly[imino(1,6-dioxohexamethylene) iminohexamethylene] (nylon 66) and (3-aminopropyl)triethoxysilane (APTES). If the tethering molecule is GA, GMBS or an activated dicarboxylic acid, a molecule containing at least one primary amine group is able to conjugate with the tethering molecule through the primary amine group in the second molecule. If the second molecule is branched and comprises multiple primary amine groups, the single second molecule is able to conjugate with multiple tethering molecules, i.e. to form multiple molecule chains connecting the substrate, providing a strong immobilization to the carbon allotrope layer.

According to some embodiments, the second molecule is identical to the first molecule.

According to some alternative embodiments, the second molecule differs from the first molecule.

For example, the second molecule is applied as an aqueous solution comprising the second molecule. For example, the concentration of the second molecule in the aqueous solution is 2 to 5 % by weight. Preferably, the second molecule is applied on the surface of the carbon allotrope layer in an amount to saturate the tethering molecule. This is to maximize the number of second molecules bonding to the tethering molecules. For example, if the tethering molecule is GA, GMBS or an activated dicarboxylic acid and the second molecule is a molecule comprising primary amine groups, the second molecule is added in such an amount that the tethering molecules are saturated with the primary amine groups of the second molecule. For example, the aqueous solution comprising the second molecule is applied on the surface of the carbon allotrope layer in an excess amount and unbonded solution is then removed from the surface.

Because the bonding of a second molecule comprising at least one primary amine group to a tethering molecule being GA, GMBS or an activated dicarboxylic acid utilizes the principles of click chemistry, the bonding takes place quickly. Therefore there is no need to keep the second molecule to be in contact with surface of the carbon allotrope layer, i.e. with the tethering molecule, for an extended period of time.

When the second molecule is a molecule containing a plurality of primary amine groups, those amine groups that are still free after bonding with the tethering molecule, are able to bond with biomolecules that are desired to be connected to the carbon allotrope layer.

### Product

The disclosure relates to a product comprising a substrate having a surface and a carbon allotrope layer connected to the surface of the substrate. For example, if the substrate is in the form of a sheet having two opposite surfaces, the carbon allotrope layer is connected to a single surface of the substrate or to both surfaces of the substrate. If the substrate has some other form, the carbon allotrope layer may be connected to the whole surface or a part of the surface.

For example, the product is can be used as a platform for a sensitive transducer for sensing probes in a biosensor and in *in-vitro* diagnostics applications.

### Substrate

The product comprises a substrate having a surface comprising functional groups. The substrate is made of solid material. The surface of the substrate is for example silicon (Si), silicon wafer, silicon dioxide (SiO₂) on silicon, quartz (SIO₂), fused quartz, aluminium Oxide (Al₂O₃), sapphire (Al₂O₃), titanium (Ti), titanium dioxide (TiO₂), glass, borofloat glass, polytetrafluoroethylene (PTFE), polyimide (PI), polycarbonate (PC), polycarbonate-polymethyl methacrylate (PC/PMMA) composite, carbon-based substrate, such as graphene, highly ordered pyrolytic graphite (HOPG), magnesium oxide (MgO), zirconium oxide (ZrO₂), or plastic film, such as polyethylene terephthalate (PET) or polystyrene (PS). For example, the substrate is in a form of a plate or a sheet.

The functional groups are selected from a group consisting of hydroxyl groups (-OH), amine groups (-NH₂) and carboxyl (-COOH) groups. In other words, the functional groups are either hydroxyl groups, amine groups or carboxyl groups, or they can be any combination of those.

### First molecule

The product comprises a first molecule, or a primer molecule, bonded to at least one of the functional groups on the surface of the substrate through covalent bonding. In other words, the product comprises a molecule formed by one of the functional groups and a first molecule bonded to the surface of the substrate. In this context, term *"first molecule"* means at least one molecule of a first molecular substance.

The first molecule adheres the carbon allotrope layer to the substrate.

Preferably, the first molecule is a polymer. According to some embodiments, the first molecule is an organosilane, such as selected from a group consisting of trimethoxymethylsilane (TMMS), dimethoxydiphenylsilane (DMDPS) and 3-(triethoxysilyl)propylmalonic acid, or the first molecule is a molecule containing at least one primary amine group, such as selected from a group consisting of (3-aminopropyl)trimethoxysilane (APTMS), bis(3-trimethoxysilylpropyl)amine (BTMSPA), 1,4-butanediol, chitosan, preferably low molecular weight chitosan, ethylenediamine (EDA), hexamethylenediamine (HMD), melamine, m-phenylenediamine, p-phenylenediamine, piperazine, polyallylamine (PAAm), branched polyethyleneimine (PEI), poly[imino(1,6-dioxohexamethylene) iminohexamethylene] (nylon 66), polyvinylamine (PVAm) and (3-aminopropyl)triethoxysilane (APTES). Preferably, the first molecule is chitosan.

### Carbon allotrope layer

The product comprises a carbon allotrope layer comprising a first surface and a second surface opposite the first surface. For example, the carbon allotrope layer acts as a transducer if the product is used as a platform for a biosensor.

For example, the carbon allotrope layer is a carbon nanotube layer or a graphene layer, such as a carbon nanotube film or a single-layer graphene film. The carbon allotrope layer may also be a vertically aligned carbon nanotube layer, i.e. a layer in which the carbon nanotubes are oriented parallel to the thickness of the layer. The carbon allotrope layer forms a mesh of carbon atoms bound to each other.

The first surface of the carbon allotrope layer is connected to the surface of the substrate with the first molecule. For example, the carbon allotrope layer is adhered to the surface of the substrate with the first molecule. The carbon allotrope may also be physically attached to the surface of the substrate with the first molecule.

Preferably, the thickness of the carbon allotrope layer is 10 to 350 nm.

### Tethering molecule

According to some embodiments, the product comprises a tethering molecule applied on the second surface of the carbon allotrope layer. In this context, term *"tethering molecule"* means at least one molecule of a molecular substance.

In these embodiments, the tethering molecule conjugates with the first molecule through the carbon allotrope layer to immobilize the carbon allotrope layer on the surface of the substrate. In other words, the tethering molecule bonds to the first molecule through the mesh formed by the carbon allotrope layer. As a result, the first molecule and the tethering molecule form a molecule chain comprising the first molecule and the tethering molecule.

In other words, the product comprises a molecule chain bonded to the substrate and extending through the carbon allotrope layer to immobilize the carbon allotrope layer on the surface of the substrate, wherein the molecule chain comprises at least one of the functional groups, the first molecule and the tethering molecule. Bonding of the tethering molecule to the first molecule through the carbon allotrope layer immobilizes the carbon allotrope layer on the surface of the substrate.

The tethering molecule may act as a hydrophilic agent, which infiltrates and surrounds the porous carbon allotrope network. This renders the external surfaces of the carbon allotrope layer hydrophilic while preserving the inherent pristine quality of the carbon allotrope layer. This enhances the electrochemical characteristics of the product without compromising the fundamental properties for its intended end-use.

Preferably, the tethering molecule is glutaraldehyde (GA). GA acts as a hydrophilic agent. More preferably, the first molecule is chitosan, and the second molecule is glutaraldehyde (GA).

### Second molecule and tethering molecule

According to some embodiments, the product comprises a second molecule applied on the second surface of the carbon allotrope layer. In this context, term *"second molecule"* means at least one molecule of a second molecular substance.

In these embodiments, the product comprises a tethering molecule. The tethering molecule conjugates with the first molecule and the second molecule through the carbon allotrope layer to immobilize the carbon allotrope layer on the surface of the substrate.

Preferably, the second molecule is a polymer. According to some embodiments, the second molecule is a polymer containing at least one primary amine group, such as selected from a group consisting of polyallylamine (PAAm), poly(amino acid), chitosan, preferably low molecular weight chitosan, branched polyethyleneimine (PEI), poly(glutamic acid), poly(L-lysine) (PLL), polyvinylamine (PVAm), poly[imino(1,6-dioxohexamethylene) iminohexamethylene] (nylon 66) and (3-aminopropyl)triethoxysilane (APTES).

The tethering molecule is selected from a group consisting of 1-ethyl-3-(3-dimethylaminopropyl)carbodiimide (EDC), N,N'-dicyclohexylcarbodiimide (DCC), glutaraldehyde (GA), N-γ-maleimidobutyryl-oxysuccinimide ester (GMBS) and activated dicarboxylic acids.

If the tethering molecule is EDC or DCC, 3-(triethoxysilyl)propylmalonic acid reacts with EDC or DCC to form a reactive O-acylisourea intermediate, which couples with a nucleophile (amine) to create an amide bond with the second molecule. EDC or DCC reacts only on the carboxylic functional group whilst the silane group reacts primarily on the activated substrate.

If the tethering molecule is GA, GMBS or an activated dicarboxylic acid, a molecule containing at least one primary amine group is able to conjugate with the tethering molecule through the primary amine group in the second molecule. If the second molecule is branched and comprises multiple primary amine groups, the single second molecule is able to conjugate with multiple tethering molecules, i.e. to form multiple molecule chains connecting the substrate, providing a strong immobilization to the carbon allotrope layer.

According to some embodiments, the second molecule is identical to the first molecule. According to some alternative embodiments, the second molecule differs from the first molecule.

The second molecule bonds to the tethering molecule through the carbon allotrope layer. In other words, the second molecule bonds to the tethering molecule through the mesh formed by the carbon allotrope layer. As a result, the first molecule, the tethering molecule and the second molecule form a molecule chain comprising the first molecule, the tethering molecule and the second molecule.

In other words, the product comprises a molecule chain bonded to the substrate and extending through the carbon allotrope layer to immobilize the carbon allotrope layer on the surface of the substrate, wherein the molecule chain comprises at least one of the functional groups, the first molecule, the tethering molecule and the second molecule. Bonding of the second molecule to the tethering molecule through the carbon allotrope layer immobilizes the carbon allotrope layer on the surface of the substrate.

This reduces the fouling and saturation of the electrode surfaces when the product is used as an electrode in a biosensor transducer. Fouling and saturation of the transducer surface leads often to surface insulation, poor performance and compromised sensitivity. This also facilitates better recognition of target analyte of interest because of a non-steric environment created by the underlying surface of the substrate covered by the porous surface of the carbon allotrope layer where it is attached.

### Use

The disclosure relates to the use of the product as described above in a biosensor. For example, the product is used as a platform for a sensitive transducer for a sensing probe in a biosensor.

### EXAMPLES

### Example 1

A polycarbonate-polymethyl methacrylate (PC/PMMA) film is provided as a substrate. The substrate is activated with plasma using a corona plasma generator. The substrate is passed under the plasma discharge generated by the corona plasma generator four times during the corona treatment to ensure consistent and homogeneous surface activation.

Within 30 minutes from the corona treatment activation, 2 % of bis(3-trimethoxysilylpropyl)amine (BTMSPA) solution is added on the activated surface of the substrate to cover the surface with the BTMSPA solution. BTMSPA solution is left on the surface of the substrate for 15 minutes.

Excess BTMSPA solution is removed from the surface of the substrate and 0.4 % glutaraldehyde (GA) solution is added on the surface covering it completely. GA solution is left on the surface of the substrate for 30 minutes.

Excess GA solution is removed from the surface of the substrate. Then, the surface of the substrate is washed with distilled water and dried with a fan to remove excess water.

Within 30 minutes of removing excess GA solution, carbon nanotube (CNT) film having a thickness of 84 nm is applied on the surface of the substrate.

Within 30 minutes of applying the CNT film on the surface of the substrate, 2 % low molecular weight chitosan solution is added on the surface of the CNT film to cover surface of the film with chitosan solution. Chitosan solution is left on the surface of film for 15 minutes.

Excess chitosan solution is removed from the surface of the film. Then, the surface of the film is washed with distilled water and dried with a fan to remove excess water.

As a result, the CNT film is immobilized on the surface of the substrate.

### Example 2

A polystyrene (PS) film is provided as a substrate. The substrate is activated with plasma using a corona plasma generator. The substrate is passed under the plasma discharge generated by the corona plasma generator four times during the corona treatment to ensure consistent and homogeneous surface activation.

Within 30 minutes from the corona treatment activation, 2 % of ethylenediamine (EDA) solution is added on the activated surface of the substrate to cover the surface with the EDA solution. EDA solution is left on the surface of the substrate for 15 minutes.

Excess EDA solution is removed from the surface of the substrate and 0.4 % glutaraldehyde (GA) solution is added on the surface covering it completely. GA solution is left on the surface of the substrate for 30 minutes.

Excess GA solution is removed from the surface of the substrate. Then, the surface of the substrate is washed with distilled water and dried with a fan to remove excess water.

Within 30 minutes of removing excess GA solution, carbon nanotube (CNT) film having a thickness of 84 nm is applied on the surface of the substrate.

Within 30 minutes of applying the CNT film on the surface of the substrate, 2 % poly(L-lysine) (PLL) solution is added on the surface of the CNT film to cover surface of the film with PLL solution. PLL solution is left on the surface of film for 15 minutes.

Excess PLL solution is removed from the surface of the film. Then, the surface of the film is washed with distilled water and dried with a fan to remove excess water.

As a result, the CNT film is immobilized on the surface of the substrate.

### Example 3

A polyethylene terephthalate (PET) film is provided as a substrate. The substrate is activated with plasma using a corona plasma generator. The substrate is passed under the plasma discharge generated by the corona plasma generator four times during the corona treatment to ensure consistent and homogeneous surface activation.

Within 30 minutes from the corona treatment activation, 2 % of low molecular weight chitosan solution is added on the activated surface of the substrate to cover the surface with the chitosan solution. Chitosan solution is left on the surface of the substrate for 15 minutes.

Excess chitosan solution is removed from the surface of the substrate and 0.4 % glutaraldehyde (GA) solution is added on the surface covering it completely. GA solution is left on the surface of the substrate for 30 minutes.

Excess GA solution is removed from the surface of the substrate. Then, the surface of the substrate is washed with distilled water and dried with a fan to remove excess water.

Within 30 minutes of removing excess GA solution, carbon nanotube (CNT) film having a thickness of 84 nm is applied on the surface of the substrate.

Within 30 minutes of applying the CNT film on the surface of the substrate, 2 % polyethyleneimine (PEI) solution is added on the surface of the CNT film to cover surface of the film with PEI solution. PEI solution is left on the surface of film for 15 minutes.

Excess PEI solution is removed from the surface of the film. Then, the surface of the film is washed with distilled water and dried with a fan to remove excess water.

As a result, the CNT film is immobilized on the surface of the substrate.

### Example 4

A polyethylene terephthalate (PET) film is provided as a substrate. The substrate is activated with plasma using a corona plasma generator. The substrate is passed under the plasma discharge generated by the corona plasma generator four times during the corona treatment to ensure consistent and homogeneous surface activation.

Within 30 minutes from the corona treatment activation, 2 % of branched polyethyleneimine (PEI) solution is added on the activated surface of the substrate to cover the surface with the PEI solution. PEI solution is left on the surface of the substrate for 15 minutes.

Excess PEI solution is removed from the surface of the substrate and 0.4 % glutaraldehyde (GA) solution is added on the surface covering it completely. GA solution is left on the surface of the substrate for 30 minutes.

Excess GA solution is removed from the surface of the substrate. Then, the surface of the substrate is washed with distilled water and dried with a fan to remove excess water.

Within 30 minutes of removing excess GA solution, carbon nanotube (CNT) film having a thickness of 84 nm is applied on the surface of the substrate.

Within 30 minutes of applying the CNT film on the surface of the substrate, 2 % branched PEI solution is added on the surface of the CNT film to cover surface of the film with PEI solution. PEI solution is left on the surface of film for 15 minutes.

Excess PEI solution is removed from the surface of the film. Then, the surface of the film is washed with distilled water and dried with a fan to remove excess water.

As a result, the CNT film is immobilized on the surface of the substrate.

### Example 5

A polystyrene (PS) film is provided as a substrate. The substrate is activated with plasma using a corona plasma generator. The substrate is passed under the plasma discharge generated by the corona plasma generator four times during the corona treatment to ensure consistent and homogeneous surface activation.

Within 30 minutes from the corona treatment activation, 2 % of 3-(triethoxysilyl)propylmalonic acid (TESPMA) solution is added on the activated surface of the substrate to cover the surface with the TESPMA solution. TESPMA solution is left on the surface of the substrate for 15 minutes.

Excess TESPMA solution is removed from the surface of the substrate and 0.4 % 1-ethyl-3-(3-dimethylaminopropyl)carbodiimide (EDC) solution is added on the surface covering it completely. EDC solution is left on the surface of the substrate for 30 minutes.

Excess EDC solution is removed from the surface of the substrate. Then, the surface of the substrate is washed with distilled water and dried with a fan to remove excess water.

Within 30 minutes of removing excess EDC solution, carbon nanotube (CNT) film having a thickness of 84 nm is applied on the surface of the substrate.

Within 30 minutes of applying the CNT film on the surface of the substrate, 2 % poly(L-lysine) (PLL) solution is added on the surface of the CNT film to cover surface of the film with PLL solution. PLL solution is left on the surface of film for 15 minutes.

Excess PLL solution is removed from the surface of the film. Then, the surface of the film is washed with distilled water and dried with a fan to remove excess water.

As a result, the CNT film is immobilized on the surface of the substrate.

## Claims

1. A method for producing a product comprising a substrate having a surface and a carbon allotrope layer connected to the surface of the substrate, **characterized in that** the method comprises
- providing a substrate having a surface,
- generating functional groups on the surface of the substrate, wherein the functional groups are selected from a group consisting of hydroxyl groups, amine groups and carboxyl groups,
- bonding a first molecule to at least one of the functional groups through covalent bonding, and
- applying a carbon allotrope layer on the surface of the substrate after bonding the first molecule to at least one of the functional groups.

2. The method according to claim 1, **characterized in that** the first molecule is an organosilane, such as selected from a group consisting of trimethoxymethylsilane (TMMS), dimethoxydiphenylsilane (DMDPS) and 3-(triethoxysilyl)propylmalonic acid.

3. The method according to claim 1, **characterized in that** the first molecule is a molecule containing at least one primary amine group, such as selected from a group consisting of (3-aminopropyl)trimethoxysilane (APTMS), bis(3-trimethoxysilylpropyl)amine (BTMSPA), 1,4-butanediol, chitosan, ethylenediamine (EDA), hexamethylenediamine (HMD), melamine, m-phenylenediamine, p-phenylenediamine, piperazine, polyallylamine (PAAm), branched polyethyleneimine (PEI), poly[imino(1,6-dioxohexamethylene) iminohexamethylene] (nylon 66), polyvinylamine (PVAm) and (3-aminopropyl)triethoxysilane (APTES).

4. The method according to claim 2 or 3, **characterized in that** the method comprises bonding a tethering molecule to the first molecule bonded to at least one of the functional groups.

5. The method according to claim 4, **characterized in that** the tethering molecule is selected from a group consisting of 1-ethyl-3-(3-dimethylaminopropyl)carbodiimide (EDC), N,N'-dicyclohexylcarbodiimide (DCC), glutaraldehyde (GA), N-γ-maleimidobutyryl-oxysuccinimide ester (GMBS) and activated dicarboxylic acids.

6. The method according to claim 4 or 5, **characterized in that** the method comprises, after applying the carbon allotrope layer on the surface of the substrate, applying the tethering molecule on the carbon allotrope layer to bond the tethering molecule to the to the first molecule through the carbon allotrope layer, wherein the first molecule is preferably chitosan, and the tethering molecule is preferably glutaraldehyde (GA).

7. The method according to claim 4 or 5, **characterized in that** the method comprises, after applying the carbon allotrope layer on the surface of the substrate, applying a second molecule on the carbon allotrope layer to bond the second molecule to the tethering molecule through the carbon allotrope layer to immobilize the carbon allotrope layer on the surface of the substrate.

8. The method according to claim 7, **characterized in that** the second molecule is a molecule containing at least one primary amine group, such as selected from a group consisting of polyallylamine (PAAm), poly(amino acid), chitosan, branched polyethyleneimine (PEI), poly(glutamic acid), poly(L-lysine) (PLL), polyvinylamine (PVAm), poly[imino(1,6-dioxohexamethylene) iminohexamethylene] (nylon 66) and (3-aminopropyl)triethoxysilane (APTES).

9. A product, **characterized in that** the product comprises
- a substrate having a surface comprising functional groups, wherein the functional groups are selected from a group consisting of hydroxyl groups, amine groups and carboxyl groups,
- a first molecule bonded to at least one of the functional groups on the surface of the substrate through covalent bonding, and
- a carbon allotrope layer comprising a first surface and a second surface opposite the first surface;
wherein the first surface of the carbon allotrope layer is connected to the surface of the substrate with the first molecule.

10. The product according to claim 9, **characterized in that** the first molecule is an organosilane, such as selected from a group consisting of trimethoxymethylsilane (TMMS), dimethoxydiphenylsilane (DMDPS), and 3-(triethoxysilyl)propylmalonic acid.

11. The product according to claim 9, **characterized in that** the first molecule is a molecule containing at least one primary amine group, such as selected from a group consisting of (3-aminopropyl)trimethoxysilane (APTMS), bis(3-trimethoxysilylpropyl)amine (BTMSPA), 1,4-butanediol, chitosan, ethylenediamine (EDA), hexamethylenediamine (HMD), melamine, m-phenylenediamine, p-phenylenediamine, piperazine, polyallylamine (PAAm), branched polyethyleneimine (PEI), poly[imino(1,6-dioxohexamethylene) iminohexamethylene] (nylon 66), polyvinylamine (PVAm) and (3-aminopropyl)triethoxysilane (APTES).

12. The product according to claim 11, **characterized in that** the product comprises a tethering molecule applied on the second surface of the carbon allotrope layer and conjugating with the first molecule through the carbon allotrope layer to immobilize the carbon allotrope layer on the surface of the substrate.

13. The product according to claim 11, **characterized in that** the product comprises
- a second molecule applied on the second surface of the carbon allotrope layer, and
- a tethering molecule conjugating with the first molecule and the second molecule through the carbon allotrope layer to immobilize the carbon allotrope layer on the surface of the substrate.

14. The product according to claim 13, **characterized in that** the tethering molecule is selected from a group consisting of 1-ethyl-3-(3-dimethylaminopropyl)carbodiimide (EDC), N,N'-dicyclohexylcarbodiimide (DCC), glutaraldehyde (GA), N-γ-maleimidobutyryl-oxysuccinimide ester (GMBS) and activated dicarboxylic acids.

15. Use of the product according to any one of claims 9 to 14 in a biosensor.
